(19) Europäisches Patentamt
European Patent Office
Office européen des brevets

(11) **EP 1 254 630 B1**

(12) **EUROPÄISCHE PATENTSCHRIFT**

(45) Veröffentlichungstag und Bekanntmachung des Hinweises auf die Patenterteilung:
**14.02.2007 Patentblatt 2007/07**

(51) Int Cl.:
***G06F 17/00*** *(2006.01)*    ***A61B 5/026*** *(2006.01)*

(21) Anmeldenummer: **02008664.1**

(22) Anmeldetag: **17.04.2002**

(54) **Vorrichtung und Computerprogramm zur Bestimmung des Blutflusses in einer Gewebe- oder Organregion**

Device and computerprogram for determination of blood flow in an area of tissue or organ

Dispositif et programme informatique pour déterminer le débit sanguin dans une région d'un tissu ou organe

(84) Benannte Vertragsstaaten:
**DE ES FR GB IT**

(30) Priorität: **01.05.2001 DE 10120980**

(43) Veröffentlichungstag der Anmeldung:
**06.11.2002 Patentblatt 2002/45**

(73) Patentinhaber: **Pulsion Medical Systems AG**
**81829 München (DE)**

(72) Erfinder:
• **Pfeiffer, Ulrich, Dr.**
**81667 München (DE)**
• **Burger, Thorsten, Dr.**
**80804 München (DE)**
• **Becker, Andreas, Dr.**
**85570 Markt Schwaben (DE)**

(74) Vertreter: **Kehl, Günther**
**Patentanwaltskanzlei**
**Günther Kehl**
**Friedrich-Herschel-Strasse 9**
**81679 München (DE)**

(56) Entgegenhaltungen:
**WO-A-98/08434**       **US-A- 5 074 306**

• **STILL J ET AL: "EVALUATION OF THE CIRCULATION OF RECONSTRUCTIVE FLAPS USING LASER-INDUCED FLUORESCENCE OF INDOCYANINE GREEN" ANNALS OF PLASTIC SURGERY, LITTLE, BROWN AND CO, US, Bd. 42, Nr. 42, 1999, Seiten 266-274, XP001063252 ISSN: 0148-7043**

**Beschreibung**

Die vorliegende Erfindung betrifft eine Vorrichtung und ein Computerprogramm zur Bestimmung des Blutflusses in einer Gewebe- oder Organregion.

Aus US 5 074 306 ist ein Verfahren zur Bestimmung von Verbrennungstiefen im Gewebe bekannt, bei dem die Fluoreszenz des exogenen Chromophors Indocyaningrün detektiert wird und anhand seiner Verteilung im Gewebe die Tiefe von Verbrennungswunden bestimmt wird.

Still J. et al : "Evaluation of the Circulation of Reconstructive Flaps using Laser-induced Fluorescence", Annals of Plastic Surgery", Little Brown and Co, US, Bd. 42, Nr. 42,1999, S. 266-274, XP001063252 ISSN: 0148-7043 beschreibt eine Vorrichtung und ein Verfahren zur Messung der Durchblutung von transferierten Hautlappen , wobei nach Applikation von Indocyaningrün die durch Bestrahlung mit einem gepulsten Laser-Array angeregte Fluoreszenz mit einer CDD-Kamera detektiert wird.

Bei den bekannten Verfahren und bekannten Vorrichtungen wird lediglich die relative Fluoreszenzverteilung des Chromophors Indocyaningrün im Gewebe qualitativ untersucht, um auf die Durchblutung des Gewebes zu schließen. Die bekannten Verfahren sind nicht geeignet, den regionalen Blutfluss anhand des Fluoreszenzsignals quantitativ zu bestimmen.

Aus WO 98 08434 A ist eine Anordnung zur nicht-invasiven Bestimmung des zerebralen Blutflusses nach intravenöser Applikation von Indocyaningrün bekannt. Die bekannte Vorrichtung umfaßt ein nicht-invasives pulsdensitometrisches Meßgerät zur Messung einer arteriellen Farbstoffkurve und zwei Nah-Infrarot-Spektroskope, die jeweils gepulste monochromatische Lichtquellen sowie einen zugehörigen Lichtaufnehmer zur gesonderten Messung der zerebralen Farbstoffkurven mit regionaler Auflösung aufweisen. Das Licht der Lichtquellen wird sowohl von Indocyaningrün als auch zum Teil von körpereigenen Chromophoren absorbiert und teilweise als Streulicht reflektiert. Letzteres wird durch die Lichtaufnehmer erfaßt und quantifiziert. Die aufgenommene Lichtmenge hängt folglich von der Absorption ab, die das Licht im Meßbereich erfährt.

In WO 96/16594 ist ein Verfahren und eine Vorrichtung zur Ermittlung der Hirndurchblutung und des intracraniellen Blutvolumens offenbart.

Aus WO 98/08434 ist ein Verfahren und eine Anordnung zur nicht-invasiven Bestimmung des cerebralen Blutflusses mittels Nah-Infrarot-Spektroskopie bekannt.

Die Verfahren der beiden zuletzt genannten Druckschriften beruhen auf der invasiven oder nicht-invasiven spektroskopischen Messung des Chromophors Indocyaningrün anhand seines Absorptionsverhaltens im Nah-Infrarot-Spektralbereich. Zudem sind diese Verfahren nicht geeignet, die zu untersuchende Gewebe- oder Organregion auf einen genau definierten Bereich einzugrenzen.

Der Erfindung liegt die Aufgabe zu Grunde, eine Vorrichtung und ein Computerprogramm zu schaffen, mit deren Hilfe der Blutfluss in einer, genau definierten, unter Umständen auch ausgedehnten, Gewebe- oder Organregion bei der routinemäßigen Anwendung im Operationssaal bequem bestimmt werden kann.

Diese Aufgabe ist gelöst mit einer Vorrichtung zur Bestimmung des Blutflusses in einer Gewebe- oder Organregion gemäß Anspruch 1.

Es wurde überraschenderweise gefunden, dass über die Berechnung der Anflutungsgeschwindigkeit eines Chromophors über die Bestimmung der Transportfunktion aus der Messung der Fluoreszenzintensität eines exogenen Chromophors der Blutfluss in einer Gewebe- oder Organregion mit sehr hoher Genauigkeit bestimmt werden kann. Gemäß der Erfindung kann beispielsweise während eines operativen Eingriffs eine Minderperfusion frühzeitig erkannt werden und noch während der Operation können korrigierende Maßnahmen eingeleitet werden. Die Erfindung kann intraoperativ einfach und ohne großen Zeitaufwand zur qualitativen Darstellung und zur Quantifizierung des Blutflusses in einem genau definierten Gewebebereich eingesetzt werden. Dabei kann eine komplette Perfusionsdarstellung in zwei Minuten durchgeführt werden. Hieraus folgt, dass die Erfindung auch während einer Operation einsetzbar ist, ohne den Ablauf der Operation zu behindern. Die Erfindung erlaubt die Aufzeichnung der Perfusion in Echtzeit in digitaler Form, gewünschtenfalls auch als Videobildsequenz. In Verbindung mit der Verwendung einer Auswerteeinheit ist eine quantitative Auswertung möglich.

Die Detektoreinheit ist eine bildgebende Einheit, die es ermöglicht, mindestens einen Bildbereich als Meßbereich und mindestens einen Bildbereich als Referenzbereich zu definieren. Zur Ermittlung der Fluoreszenzkurve, genauer gesagt, der Fluoreszenzkurven, werden nur die definierten Bereiche verwendet. Die Fluoreszenzkurve des Meßbereichs wird in Beziehung zu der Fluoreszenzkurve des Referenzbereichs gesetzt. Durch Bilden der Differenz oder des Verhältnisses der beiden Kurven können zufällige Störungen, wie beispielsweise Schwankungen der Chromophorkonzentration, bei der Berechnung des Blutflusses eliminiert werden.

Die Strahlungsquelle wird vorzugsweise so gewählt, dass sie präzise im Spektralbereich der Fluoreszenzanregung des Chromophors emittiert. Dagegen wird die Detektoreinheit, beispielsweise durch Verwendung von Filtern, auf den Spektralbereich der Fluoreszenzemission des Chromophors eingestellt, damit gesichert ist, dass nur Fluoreszenzstrahlung und nicht etwa Anregungsstrahlungin die Detektoreinheit gelangt.

Nach einem weiteren Aspekt der Erfindung bezieht sich diese auch auf ein Computerprogrammgemäß Anspruch 15.

Mit Unterstützung des Programms kann beispielsweise während eines operativen Eingriffs in kürzester Zeit ein quantitativer Wert über den Blutfluss in der zu untersuchenden Gewebe- oder Organregion ermittelt werden.

Das Computerprogramms weist ein Programmodul auf, das die Auswahl mindestens einer Meßregion und mindestens einer Referenzregion innerhalb des von der Videokamera aufgenommenen Bildes ermöglicht. Zur Auswertung wird die Fluoreszenzkurve des Meßbereichs in Beziehung zu der Fluoreszenzkurve des Referenzbereichs gesetzt. Durch Bilden der Differenz oder des Verhältnisses der beiden Kurven können zufällige Störungen, wie beispielsweise Schwankungen der Chromophorkonzentration, bei der Berechnung des Blutflusses eliminiert werden.

Eine gesteigerte Messgenauigkeit ergibt sich daraus, daß die Anflutungsgeschwindigkeit aus dem Anstieg der Transportfunktion des den Blutfluss in der Gewebe- oder Organregion bestimmenden Gefäßsystems berechnet wird. In diesem Fall ist eine präzisere quantitative Aussage über den Blutfluss möglich, insbesondere in den Fällen, in denen das Chromophor nicht "impulsartig" verabreicht werden kann. Hierzu ist nach einer besonderen Ausgestaltung des erfindungsgemäßen Computerprogramms vorgesehen, dass die Ermittlung der Transportfunktion g (t) durch mathematische Dekonvolution oder Entfaltung erfolgt indem

a) Abtastwerte des zeitlichen Verlaufs der Chromophordichte a(t) von einer Arterie stromaufwärts der Gewebe- oder Organregion ausgehend von an einem Eingang des Rechners zur Verfügung gestellten Chromophordichtemesswerten ermittelt werden und

b) die Transportfunktion g(t) durch Variation so bestimmt wird, dass der Ausdruck

$$\left[ f(t) - \int_{-\infty}^{+\infty} g(t-u)a(u)du \right]^2$$

ein Minimum annimmt.

Die erfindungsgemäße Vorrichtung und das erfindungsgemäße Computerprogramm eignen sich insbesondere zum Einsatz dort, wo die Quantifizierung der Gewebedurchblutung während eines Eingriffs entscheidend sein kann, d. h. im Bereich der Viszeralchirugie bei Linkskolon- und Rektumresektionen, bei Schlauchmagentransposition nach Ösophagussesektion, bei freien Dünndarmtransplantaten zur Interposition sowie allen Roux-Y-Rekonstruktionen (nach Gastrektomie, als biliodigestive Anastomosen etc.). Die Erfindung eignet sich auch zur Erkennung sekundärer Perfusionsstörungen bei inkarzerierter Hernie oder bei Bridenileus. In der Herzchirugie kann die Erfindung zur Überprüfung der Effizienz koronarer Bypässe eingesetzt werden. Im Bereich der plastischen Chirugie ist die Kontrolle der Perfusion transferierter Lappen, sowie die Beurteilung des Gewebeschadens bei Traumata (z. B. Navikularfrakturen, Mehrfragmentfrakturen, Weichteilverletzungen sowie Schussverletzungen) möglich.

Weitere vorteilhafte Ausführungsformen der Erfindung, sind den abhängigen Ansprüchen zu entnehmen.

Die Erfindung wird nachfolgend anhand eines in den Zeichnungen dargestellten Ausführungsbeispiels näher erläutert. Hierbei zeigt:

Figur 1: Die schematische Anordnung einer erfindungsgemäßen Vorrichtung bei der intraoperativen Verwendung,

Figur 2: Eine Grafik zur Darstellung der Fluoreszenzintensität als Funktion der Zeit.

Figur 3: Ein Sucherbild einer im Zusammenhang mit der Erfindung verwendeten Kamera

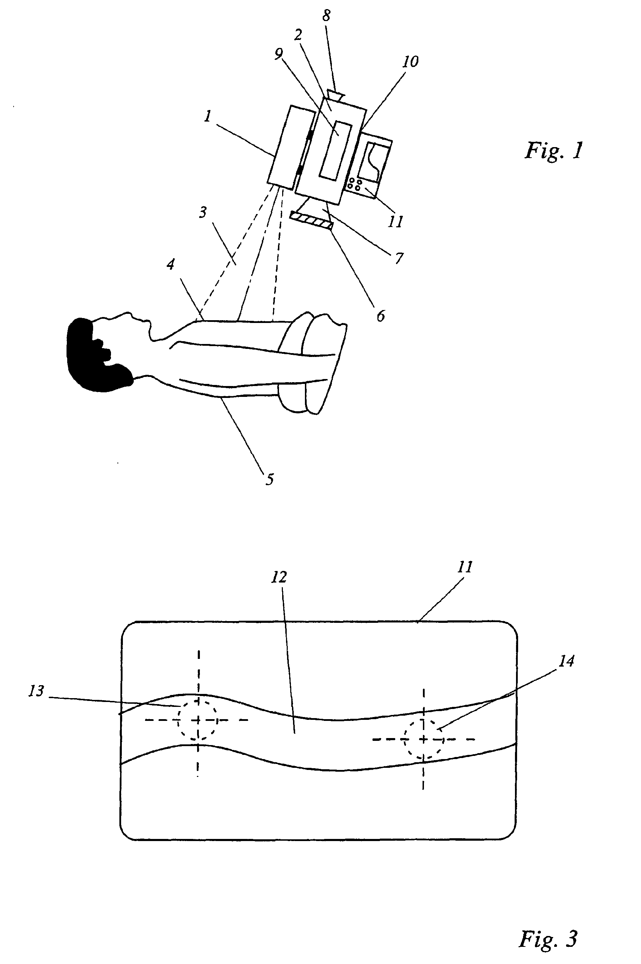

In Figur 1 ist schematisch und nicht maßstäblich eine erfindungsgemäße Vorrichtung im intraoperativen Einsatz dargestellt. Ein Sicherheitsgehäuse 1, in das eine Infrarot-Laser-Lichtquelle mit einer Peakemission von 780 nm integriert ist, bildet zusammen mit einer CCD-Kamera 2, und einer Rechen- oder Auswerteeinheit 11 eine kompakte, einhändig tragbare und bedienbare Einheit, die über einen Akkumulator verfügt und daher netzunabhängig einsetzbar ist.

Das aus dem Sicherheitsgehäuse 1 austretende aufgeweitete Laserlicht 3 hat eine flächenbezogene Intensität von weniger als 1 mW/cm$^2$ und liegt unter dem Grenzwert der maximal zulässigen Bestrahlung der Hornhaut des Auges, wodurch in der Umgebung der Vorrichtung keine Schutzbrillen getragen werden müssen.

Das aufgeweitete Strahlbündel 3 der Infrarot-Laser-Lichtquelle bestrahlt das etwa 30 cm breite Operationsfeld 4, das sich etwa in 70 cm Entfernung von dem Sicherheitsgehäuse 1 befindet. Dem Patienten 5 zuvor in einer Dosis 0,1 bis 2 mg pro kg Köpergewicht verabreichtes Indocyaningrün wird durch die Bestrahlung der Fluoreszenz angeregt.

Das Fluoreszenzsignal wird von der im Nah-Infrarot-Wellenlängenbereich empfindlichen CCD-Kamera 2 detektiert, der ein Filter 6 vorgeschaltet ist. Das Filter 6 ist ein NIR-Langpassfilter (Kantenfilter), das nur für Wellenlängen größer 800 nm durchlässig ist und mittels eines Außengewindes mit dem Autofokus-Objektiv 7 der CDD-Kamera 2 verschraubt ist. Alternativ eignet sich auch ein Filter, das eine schmalbandige Transmission im Bereich des Fluoreszenzmaximums des Chromophors Indocyaningrün erlaubt. Die CDD-Kamera 2 besitzt einen Sucher 8, so dass während der Operation kein externer Monitor eingesetzt werden muss, und somit eine möglicherweise die Handhabung beeinträchtigende Kabelverbindung entfällt. Die elektronischen Bilddaten der aufgenommenen Fluoreszenz werden auf einer Videokasette 9 digital aufgezeichnet.

Zwischen dem Patienten 5 und der Einheit aus CDD-Kamera 2 und Sicherheitsgehäuse 1 kann ein steriles Tuch (nicht dargestellt) angeordnet werden, so dass die Vorrichtung selbst nicht steril sein muss. Auf Grund der kompakten Bauweise und des Wegfallens von Kabelverbindungen kann die Einheit aus CCD-Kamera 2 und Sicherheitsgehäuse 1 aber auch leicht steril verpackt werden.

Über ein Schnittstelle 10 nach IEEE 1394, die eine Datenübertragungsrate von bis zu 400 MBit/s erlaubt, kann ein elektronisches Bildverarbeitungs- und -auswertesystem 11 an die CCD-Kamera 2 angeschlossen werden, die es erlaubt, die Helligkeit der einzelnen Bildelemente (Pixel) quantitativ als Maß für die Fluoreszenzintensität zu erfassen. Hierzu können auf dem ersten Bild einer Bildsequenz durch den Benutzer verschiedene Bildbereiche markiert werden, um dann Bild für Bild die Helligkeit der Pixel in diesem Areal zu ermitteln und die Ergebnisse graphisch darzustellen. Dabei kann eine zu untersuchende Gewebe- oder Organregion direkt mit einem Referenzareal mit normaler Perfusion oder aber mit einem externen Standard bekannter Intensität verglichen werden. Bei der Verwendung eines externen Standards können auch Bildsequenzen die mit unterschiedlichen Bestrahlungs- oder Detektorparametern aufgenommen wurden, direkt miteinander verglichen werden. Durch die Auswertung der gesamten Bildsequenz ist es möglich, verschiedene Kriterien wie z. B. die Geschwindigkeit der Anflutung und Abflutung des Chromophors und die durch das Chromophor verursachte Veränderung der Fluoreszenzintensität in den Gewebearealen zur Auswertung heranzuziehen.

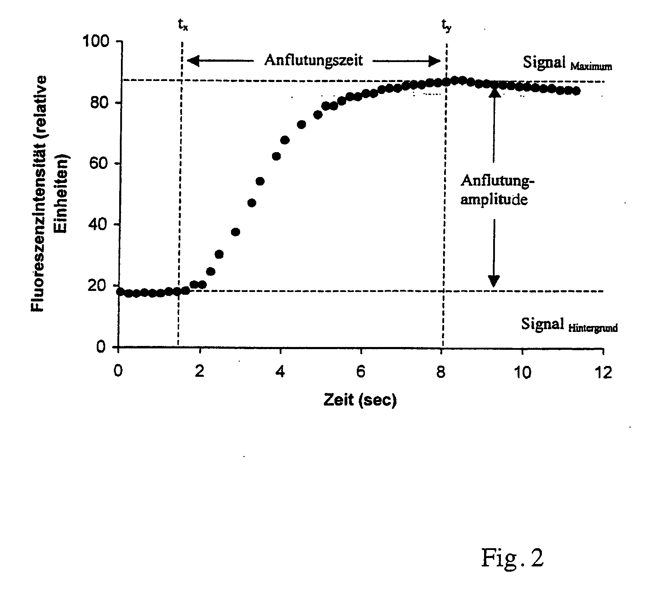

Figur 2 zeigt in einer graphischen Darstellung die Fluoreszenzintensität (gemessen in relativen Einheiten von 0 -100) als Funktion der Zeit (gemessen in Sekunden). Es ist zu erkennen, dass die Kurve der Fluoreszenzintensität von einem Hintergrundsignal von 20 Einheiten ausgeht und beginnend mit dem Zeitpunkt $t_x$ rasch ansteigt, bis das Signalmaximum zum Zeitpunkt $t_y$ (8 Sekunden) erreicht ist. Danach bleibt das Fluoreszenzintensitätssignal konstant und fällt später langsam ab. Der Zeitraum zwischen den Zeitpunkten $t_x$ und $t_y$, der als Anflutungszeit bezeichnet werden kann, ermöglicht die Berechnung des Blutflusses in der untersuchten Gewebe- oder Organregion. Das bedeutet, dass der Blutfluss umso größer ist, je kürzer die Anflutungszeit ist und folglich je steiler die Fluoreszenzkurve verläuft.

Aus der Fluoreszenzkurve, die direkt die gemessene Fluoreszenzintensität in Abhängigkeit der Zeit wiedergibt, kann durch mathematische Entfaltung eine Transportfunktion gewonnen werden. Die Transportfunktion entspricht einer theoretischen Fluoreszenzkurve, die erhalten worden wäre, wenn das Chromophor vollständig zu einem einzelnen Zeitpunkt (ohne zeitliche Ausdehnung) verabreicht worden wäre. Durch Berechnung der Transportfunktion werden folglich alle in der Praxis auftretenden Zufälligkeiten, die dadurch verursacht werden, dass das Chromophor nicht schlagartig, sondern langsam über einen längeren Zeitraum verabreicht wird, ausgeschaltet . Die durch Entfaltung gewonnene Transportfunktion weist qualitativ den gleichen Verlauf wie die in Figur 2 gezeigte Fluoreszenzkurve auf, verläuft jedoch etwas steiler.

Figur 3 zeigt ein Sucherbild der bei der Erfindung als Detektoreinheit verwendeten Kamera. Im Zentrum des Sucherbildes ist schematisch ein Schlauchmagentransponat zum Ösophagusersatz gezeigt. Zwei Zielfadenkreuze 13 und 14 können über Bedienknöpfe (nicht dargestellt) auf bestimmte Regionen von Interesse innerhalb des Sucherbildes gerichtet werden. Das Zielfadenkreuz 13 ist beispielsweise auf eine hinsichtlich der Durchblutung besonders kritische Stelle des Transponats gerichtet, während das Zielfadenkreuz 14 auf eine Stelle des Transponats gerichtet ist, deren gute Durchblutung gesichert ist. Beim Betrieb der Detektoreinheit 2 werden nur die Bildpunkte (Pixel) innerhalb der durch die Fadenkreuze ausgewählten Regionen verwertet und zwar die Region im Zielfadenkreuz 13 als Meßregion und die Region im Zielfadenkreuz 14 als Referenzregion. Auf diese Weise kann der Blutfluss in der Meßregion des Zielfadenkreuzes 14 quantitativ bestimmt werden, wobei Zufälligkeiten, wie beispielsweise Schwankungen in der Chromophorkonzentration etc., durch die Differenzbildung zu der Messung in der Referenzregion eliminiert werden. Alternativ oder ergänzend zu der Auswahl einer Geweberegion, die als Referenzregion dient, kann auch ein externer Referenzstandard ausgewählt werden

**Patentansprüche**

1. Vorrichtung zur Bestimmung des Blutflusses in einer Gewebe- oder Organregion,

   (a) mit einer Bestrahlungseinheit (1) zur Bestrahlung der Gewebe- oder Organregion mit elektromagnetischer Strahlung in einem bestimmten Spektralbereich,
   (b) mit einer Detektoreinheit (2), die eine bildgebende Einrichtung zur Messung einer Fluoreszenzintensität eines fluoreszierenden Chromophors in der Gewebe- oder Organregion zur Bestimmung einer Fluoreszenzkurve f(t) umfaßt, **gekennzeichnet durch**
   (c) eine Auswahleinheit, mit der in dem von der bildgebenden Einrichtung erzeugten Bild mindestens eine Meßregion (13) und mindestens eine Referenzregion (14) und/oder ein externer Referenzstandard auswählbar sind, und
   (d) eine Recheneinheit (11), die ausgelegt ist, die Anflutungsgeschwindigkeit des Chromophors in der Meßregion (13) unter Verwertung der Fluoreszenzkurve zu berechnen, indem die Transportfunktion g(t) des für den Blutfluß in der Gewebe- oder Organregion maßgeblichen Gefäßsystems **durch** mathematische Dekonvolution berechnet und die Anflutungsgeschwindigkeit aus der Steilheit des Anstiegs der Transportfunktion g(t) bestimmt wird, und den Blutfluss aus der berechneten Anflutungsgeschwindigkeit zu bestimmen,

welche Recheneinheit (11) ferner ausgelegt ist, um bei der Bestimmung des Blutflusses die Anflutungsgeschindigkeit mit den Ergebnissen einer parallelen Messung in der Referenzregion (14) und/oder mit dem externen Referenzstandard in Beziehung zu setzen.

2. Vorrichtung nach Anspruch 1, **dadurch gekennzeichnet, dass** die bildgebende Einrichtung eine digitale Kamera (2) ist.

3. Vorrichtung nach Anspruch 2, **dadurch gekennzeichnet, dass** die digitale Kamera (2) zur Aufzeichnung einer Videobildsequenz von der Gewebe- oder Organregion ausgelegt ist.

4. Vorrichtung nach einem der vorhergehenden Ansprüche , **dadurch gekennzeichnet, dass** die Strahlungsquelle (1) ausgelegt ist, Strahlung im Spektralbereich des Absorptionsmaximums des Chromophors zu emittieren.

5. Vorrichtung nach einem der vorhergehenden Ansprüche, **dadurch gekennzeichnet, dass** die Strahlungsquelle (1) eine Leuchtdiodeneinheit mit gebündelter Emission ist.

6. Vorrichtung nach einem der vorhergehenden Ansprüche , **dadurch gekennzeichnet, dass** die Strahlungsquelle (1) eine Infrarot-Laser-Lichtquelle ist.

7. Vorrichtung nach einem der vorhergehenden Ansprüche , **dadurch gekennzeichnet, dass** die Strahlungsquelle (1) eine Laserdiode ist.

8. Vorrichtung nach einem der vorhergehenden Ansprüche, **dadurch gekennzeichnet, dass** die Strahlungsquelle (1) gepulst ist.

9. Vorrichtung nach einem der vorhergehenden Ansprüche, **dadurch gekennzeichnet, dass** die Strahlungsquelle (1) moduliert ist.

10. Vorrichtung nach einem der vorhergehenden Ansprüche, **dadurch gekennzeichnet, dass** die Strahlungsquelle (1) eine flächenbezogene Intensität von weniger als $1mW/cm^2$ aufweist und ungefährlich für das ungeschützte menschliche Auge ist.

11. Vorrichtung nach einem der vorhergehenden Ansprüche, **dadurch gekennzeichnet, dass** die Strahlungsquelle ausgelegt ist, im Spektralbereich um 805 nm zu emittieren.

12. Vorrichtung nach einem der vorhergehenden Ansprüche, **dadurch gekennzeichnet, dass** die Detektoreinheit ausgelegt ist, Strahlung im Bereich der Fluoreszenzwellenlänge des Chromophors zu detektieren.

13. Vorrichtung nach einem der vorhergehenden Ansprüche, **dadurch gekennzeichnet, dass** die Detektoreinheit ausgelegt ist, Strahlung im Bereich von 835 nm zu detektieren.

14. Vorrichtung nach einem der vorhergehenden Ansprüche , **dadurch gekennzeichnet, dass** sie als eine kompakte Einheit ausgebildet ist.

15. Computerprogramm, insbesondere auf ein Speichermedium aufgezeichnetes Computerprogramm, das in den Programmspeicher eines Rechners zu laden ist und den Rechner veranlasst, folgende Schritte auszuführen:

(a) Erzeugung von Abtastwerten einer Fluoreszenzkurve f(t) ausgehend von an einem Eingang des Rechners zur Verfügung gestellten Fluoreszenzintensitätsmesswerten,
(b) Auswahl der Abtastwerte aus mindestens einer Meßregion (14) und mindestens einer Referenzregion (13) und/oder Auswahl eines externen Referenzstandards mittels eines Programmoduls, ausgehend von durch eine Auswahleinheit definierten Bereichen,
(c) Berechnung der Anflutungsgeschwindigkeit eines Chromophors in der Meßregion (13) aus den Abtastwerten der Fluoreszenzkurve f(t) aus der ausgewählten Region bzw. den ausgewählten Regionen, indem die Transportfunktion g(t) des für den Blutfluß in der Gewebe- oder Organregion maßgeblichen Gefäßsystems durch mathematische Dekonvolution berechnet und die Anflutungsgeschwindigkeit aus der Steilheit des Anstiegs der Transportfunktion g(t) bestimmt wird,.
(d) Bestimmung des Blutflusses aus der berechneten Anflutungsgeschwindigkeit, wobei die Anflutungsgeschwindigkeit mit den Ergebnissen einer parallelen Messung in der Referenzregion (14) und/oder mit dem externen Referenzstandard in Beziehung gesetzt wird.

16. Computerprogramm nach Anspruch 15, das folgende Schritte enthält:

(a) Bestimmen von Abtastwerten des zeitlichen Verlaufs der Chromophordichte a(t) ausgehend von an einem Eingang des Rechners zur Verfügung gestellten Chromophordichtemesswerten von einer Arterie stromaufwärts der Gewebe- oder Organregion und

(b) Bestimmen der Transportfunktion g(t) durch mathematische Dekonvolution, so dass der Ausdruck

$$[f(t) - \int_{-\infty}^{+\infty} g(t-u)a(u)du]^2$$

ein Minimum annimmt.

**Claims**

1. Device for determining the blood flow in a tissue or organ region,

   (a) comprising an irradiation unit (1) for irradiating the tissue or organ region with electromagnetic radiation in a specific spectral range,
   (b) comprising a detector unit (2), which comprises an imaging mechanism for measuring a fluorescence intensity of a fluorescent chromophore in the tissue or organ region for determining a fluorescence curve f(t)
   **characterised by**
   (c) a selection unit with which, in the image generated by the imaging mechanism, at least one measuring region (13) and at least one reference region (14) and/or an external reference standard can be selected, and
   (d) an arithmetic unit (11), which is designed to calculate the permeation rate of the chromophore in the measuring region (13) by using the fluorescence curve, in which the transport function g(t) of the vascular system, which is decisive for the blood flow in the tissue or organ region, is calculated by mathematical deconvolution and the permeation rate is determined from the steepness of the rise in the transport function g(t), and to determine the blood flow from the calculated permeation rate,

   which arithmetic unit (11) is also designed, during the determination of the blood flow, to relate the permeation rate to the results of a parallel measurement in the reference region (14) and/or to the external reference standard.

2. Device according to claim 1, **characterised in that** the imaging mechanism is a digital camera (2).

3. Device according to claim 2, **characterised in that** the digital camera (2) is designed to record a video image sequence of the tissue or organ region.

4. Device according to any one of the preceding claims, **characterised in that** the radiation source (1) is designed to emit radiation in the spectral range of the absorption maximum of the chromophore.

5. Device according to any one of the preceding claims, **characterised in that** the radiation source (1) is a light-emitting diode unit with bundled emission.

6. Device according to any one of the preceding claims, **characterised in that** the radiation source (1) is an infrared laser light source.

7. Device according to any one of the preceding claims, **characterised in that** the radiation source (1) is a laser diode.

8. Device according to any one of the preceding claims, **characterised in that** the radiation source (1) is pulsed.

9. Device according to any one of the preceding claims, **characterised in that** the radiation source (1) is modulated.

10. Device according to any one of the preceding claims, **characterised in that** the radiation source (1) has a surface intensity of less than 1 mW/cm$^2$ and is harmless to the unprotected human eye.

11. Device according to any one of the preceding claims, **characterised in that** the radiation source is designed to emit in the spectral range around 805 nm.

12. Device according to any one of the preceding claims, **characterised in that** the detector unit is designed to detect radiation in the range of the fluorescence wavelength of the chromophore.

13. Device according to any one of the preceding claims, **characterised in that** the detector unit is designed to detect radiation in the range of 835 nm.

14. Device according to any one of the preceding claims, **characterised in that** it is configured as a compact unit.

15. Computer program, in particular a computer program recorded on a storage medium, which computer program is to be loaded into the program memory of a computer and causes the computer to carry out the following steps:

   (a) generation of sample values of a fluorescence curve f(t) proceeding from fluorescence intensity measured values provided at an input of the computer,
   (b) selection of the sample values from at least one measuring region (14) and at least one reference region (13) and/or selection of an external reference standard by means of a program module proceeding from regions defined by a selection unit,

(c) calculation of the permeation rate of a chromophore in the measuring region (13), from the sample values of the fluorescence curve f(t) from the selected region or the selected regions, in which the transport function g(t) of the vascular system, which is decisive for the blood flow in the tissue or organ region, is calculated by mathematical deconvolution and the permeation rate is determined from the steepness of the rise in the transport function g(t),

(d) determination of the blood flow from the calculated permeation rate, the permeation rate being related to the results of a parallel measurement in the reference region (14) and/or to the external reference standard.

16. Computer program according to claim 15, which contains the following steps:

(a) determination of sample values of the time course of the chromophore density a(t) proceeding from chromophore density measured values, which are provided at an input of the computer, from an artery upstream from the tissue or organ region and

(b) determination of the transport function g(t) by mathematical deconvolution, so the expression

$$[f(t)- \int_{-00}^{+00} g\,(t\text{-}u)a(u)du]^2$$

assumes a minimum.

**Revendications**

1. - Dispositif pour la détermination du flux sanguin dans une région de tissu ou d'organe,

(a) ayant une unité d'irradiation (1) pour l'irradiation de la région de tissu ou d'organe par un rayonnement électromagnétique dans une région spectrale définie ;

(b) ayant une unité détecteur (2), qui comprend un dispositif de production d'image pour la mesure d'une intensité de fluorescence d'un chromophore fluorescent dans la région de tissu ou d'organe pour la détermination d'une courbe de fluorescence f(t),

**caractérisé par**

(c) une unité de sélection, avec laquelle dans l'image produite par le dispositif de production d'image, sont aptes à être choisies au moins une région de mesure (13) et au moins une région de référence (14) et/ou un standard de référence externe ; et

(d) une unité de calcul (11), qui est agencée pour calculer la vitesse d'afflux du chromophore dans la région de mesure (13) sous utilisation de la courbe de fluorescence, en ce que la fonction de transport g(t) du système vasculaire qui sert de norme pour le flux sanguin dans la région de tissu ou d'organe est calculée par déconvolution mathématique et la vitesse d'afflux est déterminée à partir de la pente de la montée de la fonction de transport g(t), et pour déterminer le flux sanguin à partir de la vitesse d'afflux calculée ;

laquelle unité de calcul (11) est agencée en outre pour, lors de la détermination du flux sanguin, mettre en rapport la vitesse d'afflux avec les résultats d'une mesure parallèle dans la région de référence (14) et/ou avec le standard de référence externe.

2. - Dispositif selon la revendication 1, **caractérisé par le fait que** le dispositif de production d'image est une caméra numérique (2).

3. - Dispositif selon la revendication 2, **caractérisé par le fait que** la caméra numérique (2) est agencée pour enregistrer une séquence d'images vidéo de la région de tissu ou d'organe.

4. - Dispositif selon l'une des revendications précédentes, **caractérisé par le fait que** la source de rayonnement (1) est agencée pour émettre un rayonnement dans la région spectrale du maximum d'absorption du chromophore.

5. - Dispositif selon l'une des revendications précédentes, **caractérisé par le fait que** la source de rayonnement (1) est une unité de diodes luminescentes avec une émission en faisceau.

6. - Dispositif selon l'une des revendications précédentes, **caractérisé par le fait que** la source de rayonnement (1) est une source de lumière laser infrarouge.

7. - Dispositif selon l'une des revendications précédentes, **caractérisé par le fait que** la source de rayonnement (1) est une diode laser.

8. - Dispositif selon l'une des revendications précédentes, **caractérisé par le fait que** la source de rayonnement (1) est pulsée.

9. - Dispositif selon l'une des revendications précédentes, **caractérisé par le fait que** la source de rayonnement (1) est modulée.

**10.** - Dispositif selon l'une des revendications précédentes, **caractérisé par le fait que** la source de rayonnement (1) présente une intensité surfacique de moins de 1 mW/cm$^2$ et est sans danger pour l'oeil humain non protégé.

**11.** - Dispositif selon l'une des revendications précédentes, **caractérisé par le fait que** la source de rayonnement est agencée pour émettre dans la région spectrale autour de 805 nm.

**12.** - Dispositif selon l'une des revendications précédentes, **caractérisé par le fait que** l'unité détecteur est agencée pour détecter un rayonnement dans la région de la longueur d'onde de fluorescence du chromophore.

**13.** - Dispositif selon l'une des revendications précédentes, **caractérisé par le fait que** l'unité détecteur est agencée pour détecter un rayonnement dans la région de 835 nm.

**14.** - Dispositif selon l'une des revendications précédentes, **caractérisé par le fait qu'**il est réalisé sous la forme d'une unité compacte.

**15.** - Programme d'ordinateur, en particulier programme d'ordinateur enregistré sur un support d'enregistrement, qui est à charger dans la mémoire de programme d'un calculateur et amène le calculateur à exécuter les étapes suivantes :

    (a) génération à partir de valeurs de balayage d'une courbe de fluorescence f(t) à partir de valeurs d'intensité de fluorescence mises à disposition sur une entrée du calculateur ;
    (b) sélection des valeurs de balayage à partir d'au moins une région de mesure (14) et d'au moins une région de référence (13) et/ou sélection d'un standard de référence externe au moyen d'un module de programme, à partir de zones définies par une unité de sélection ;
    (c) calcul de la vitesse d'afflux d'un chromophore dans la région de mesure (13) à partir des valeurs de balayage de la courbe de fluorescence f(t) à partir de la région sélectionnée ou des régions sélectionnées, en ce que la fonction de transport g(t) du système vasculaire qui sert de norme pour le flux sanguin dans la région du tissu ou d'organe est calculée par déconvolution mathématique et la vitesse d'afflux est déterminée à partir de la pente de la courbe de la montée de la fonction de transport g(t) ;
    (d) détermination du flux sanguin à partir de la vitesse d'afflux calculée, à l'occasion de quoi la vitesse d'afflux est mise en rapport avec les résultats d'une mesure parallèle dans la région de référence (14) et/ou avec le standard de référence externe.

**16.** - Programme d'ordinateur selon la revendication 15, qui contient les étapes suivantes :

    (a) détermination de valeurs de balayage de la variation dans le temps de l'intensité de chromophore a(t) à partir de valeurs d'intensité de chromophore mises à disposition sur une entrée du calculateur d'une artère en amont de la région de tissu ou d'organe ; et
    (b) détermination de la fonction de transport g(t) par déconvolution mathématique de telle sorte que l'expression

$$\left[ f(t) - \int_{-\infty}^{+\infty} g(t-u)a(u)du \right]^2$$

admette un minimum.

*Fig. 1*

*Fig. 3*

EP 1 254 630 B1

Fig. 2